# EUROPEAN PATENT APPLICATION

(11) **EP 3 505 146 A1**
(43) Date of publication of application: **03.07.2019**
(21) Application number: 17843711.7
(22) Date of filing: 25.08.2017
(51) Int. Cl.: A61F 11/00, G09B 5/04

(54) **AUDITORY TRAINING DEVICE, AUDITORY TRAINING METHOD, AND PROGRAM**

(30) Priority: 25.08.2016 JP 2016164524
(71) Applicant: Frontiermarketsjapan, Inc., Tokyo 111-0034 (JP)
(72) Inventor: SUWA, Kanehisa, Tokyo 111-0042 (JP)
(74) Representative: Bandpay & Greuter
(86) International application number: PCT/JP2017/030433
(87) International publication number: WO 2018/038235

(57) **Abstract**

Hearing training apparatus 1 includes acquisition means 11 that acquires target audio, determination means 14 that determines an attribute of a noise to be added to the target audio according to a provided training intensity, addition means 15 that adds a noise having an attribute determined by determination means 14 to the target audio acquired by acquisition means 12, and output means 16 that outputs the target audio together with the noise added to the examination audio by addition means 15.

## Description

### TECHNICAL FIELD

The present invention relates to technology for training hearing.

### BACKGROUND

Technology for training the brain using sounds is known. For example, Patent Document 1 discloses technology that promotes brain activity by outputting a sound in a tone range together with a sound in a different band.

### Citation List

### Patent Literature

Patent Document 1: JP 2015-157118A

### SUMMARY

### TECHNICAL PROBLEM

The technology disclosed in Patent Document 1 has a problem in that brain stimuli are monotonous.

In contrast, the present invention provides hearing training technology in which training intensity can be changed.

### SOLUTION

The present invention provides a hearing training apparatus that includes an acquisition means that acquires examination audio in which sounds selected from a determined correct sound group and a determined wrong sound group are arranged in a time sequence, a determination means that determines an attribute of at least one of the examination audio and a noise to be added to the examination audio according to a provided training intensity, an addition means that adds a noise having an attribute determined by the determination means to the examination audio, and an output means that outputs the examination audio together with the noise added to the examination audio by the addition means.

The hearing training apparatus may include an accepting means that accepts input of an answer regarding a sound that belongs to the wrong sound group out of sounds presented by the examination audio.

The accepting means may accept, as the answer, the number of sounds that belong to the wrong sound group out of the sounds presented by the examination audio.

The accepting means may accept, as the answer, a character string that represents the sound belonging to the wrong sound group out of the sounds presented by the examination audio.

The accepting means may accept, as the answer, a time at which the sound belonging to the wrong sound group is output while the examination audio is being output by the output means.

The hearing training apparatus may include a storage means that stores audio fragments and an audio synthesis means that synthesizes the examination audio using the audio fragments, and the acquisition means may acquire the examination audio synthesized by the audio synthesis means.

The audio synthesis means may set a frequency of audio in which a sound belonging to the wrong sound group is spoken to be higher than a frequency of audio in which a sound belonging to the correct sound group is spoken.

The storage means may store audio fragments of a plurality of speakers, and the audio synthesis means may synthesize the examination audio using audio fragments of a speaker who is selected from among the plurality of speakers according to the training intensity.

The storage means may store a plurality of word groups each including a plurality of words, the hearing training apparatus may include a selection means that selects a correct word group and a wrong word group from among the plurality of word groups, and the audio synthesis means may synthesize audio in which words selected from the correct word group and the wrong word group are spoken in order on a time axis.

The attribute determined by the determination means may include at least one of a noise sound source, volume, a relative playback speed of the noise relative to the examination audio, and the number of noise sound sources.

The present invention also provides a hearing training method including steps of acquiring examination audio in which sounds selected from a determined correct sound group and a determined wrong sound group are spoken in order, determining an attribute of at least one of the examination audio and a noise to be added to the examination audio according to a provided training intensity, adding a noise having an attribute determined by the determination means to the examination audio, and outputting the examination audio together with the noise added to the examination audio.

The present invention further provides a program that causes a computer to execute steps of acquiring examination audio in which sounds selected from a determined correct sound group and a determined wrong sound group are spoken in order, determining an attribute of at least one of the examination audio and a noise to be added to the examination audio according to a provided training intensity, adding a noise having an attribute determined by the determination means to the examination audio, and outputting the examination audio together with the noise added to the examination audio.

The present invention also provides a hearing training apparatus including an acquisition means that acquires target audio, a storage means that stores a level of performance that indicates progress of a hearing training of a user, an acquisition means that acquires training intensity of the user calculated using the level of performance stored in the storage means, a determination means that automatically determines an attribute of a noise to be added to the target audio according to the training intensity acquired by the acquisition means, an addition means that adds the noise having the attribute determined by the determination means to the target audio acquired by the acquisition means, and an output means that outputs the target audio together with the noise added to the target audio by the addition means.

The attribute determined by the determination means may include at least one of a noise sound source, volume, a relative playback speed of the noise relative to the target audio, and the number of noise sound sources.

The determination means may determine the attribute of the noise according to an attribute of the user who performs the training, in addition to the training intensity.

The hearing training apparatus may include an accepting means that accepts feedback from the user regarding the target audio output by the output means, and the determination means may use the feedback obtained from the user as the attribute of the user.

The present invention also provides an operation method of a hearing training apparatus including steps of acquiring target audio by the hearing training apparatus, storing, by the hearing training apparatus, a level of performance that indicates progress of a hearing training of a user in a storage means, acquiring, by the hearing training apparatus, training intensity of the user calculated using the level of performance stored in the storage means, automatically determining, by the hearing training apparatus, an attribute of a noise to be added to the target audio according to the acquired training intensity, adding, by the hearing training apparatus, the noise having the determined attribute to the acquired target audio, and outputting, by the hearing training apparatus, the target audio together with the noise added to the target audio.

The present invention further provides a program that causes a computer to execute steps of acquiring target audio, storing a level of performance that indicates progress of a hearing training of a user in a storage means, acquiring training intensity of the user calculated using the level of performance stored in the storage means, automatically determining an attribute of a noise to be added to the target audio according to the acquired training intensity, adding the noise having the determined attribute to the acquired target audio, and outputting the target audio together with the noise added to the target audio.

### Advantageous Effects of Invention

According to the present invention, training intensity can be changed in hearing training technology.

### BRIEF DESCRIPTION OF DRAWINGS

FIG. 1 is a diagram illustrating an example of a functional configuration of hearing training apparatus 1 according to a first embodiment.
FIG. 2 is a diagram illustrating an example of a hardware configuration of hearing training apparatus 1.
FIG. 3 is a sequence chart illustrating an example of operations of hearing training apparatus 1.
FIG. 4 is a diagram illustrating an example of UI screen 40 for accepting an instruction to start training.
FIG. 5 is a diagram illustrating an example of UI screen 50 for accepting feedback.
FIGS. 6 are diagrams illustrating an example of feedback from a user.
FIG. 7 is a diagram illustrating an example of a functional configuration of hearing training apparatus 2 according to a second embodiment.
FIG. 8 is a sequence chart illustrating an example of examination audio synthesis processing according to the second embodiment.
FIG. 9 is a diagram illustrating an example of UI screen 60 for accepting an instruction to start training.
FIG. 10 is a diagram illustrating an example of a plurality of sound groups.
FIG. 11 is a diagram illustrating an example of examination audio.

### REFERENCE SIGNS LIST

1 ... Hearing training apparatus, 11 ... Acquisition means, 12 ... Acquisition means, 13 ... Storage means, 14 ... Determination means, 15 ... Addition means, 16 ... Output means, 17 ... Accepting means, 21 ... Selection means, 22 ... Audio synthesis means, 101 ... CPU, 102 ... Memory, 103 ... Storage, 104 ... Touch screen, 105 ... Microphone, 106 ... Headphone

### DESCRIPTION OF EMBODIMENTS

### 1. First Embodiment

### 1-1. Configuration

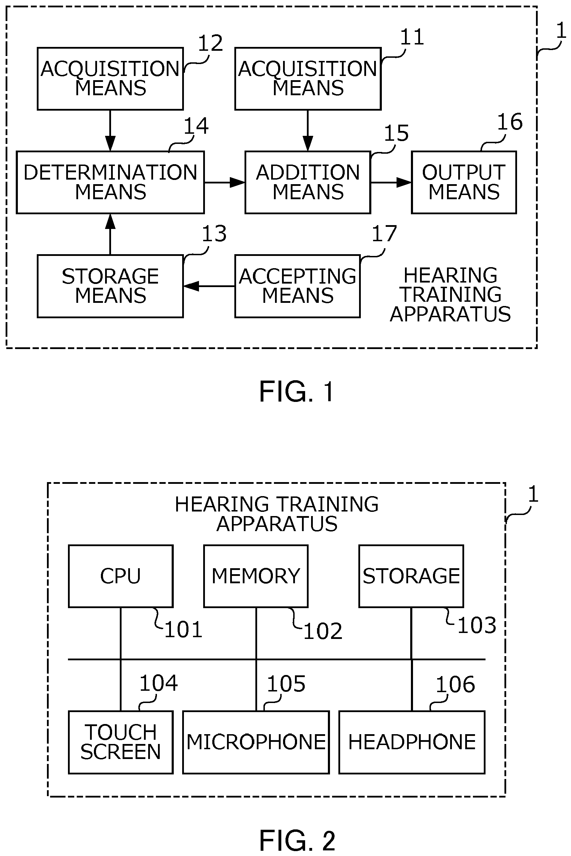

FIG. 1 is a diagram illustrating an example of a functional configuration of hearing training apparatus 1 according to a first embodiment. A hearing training apparatus is an apparatus that stimulates the brain through hearing to improve hearing or suppress the progression of dementia, for example.

Hearing training apparatus 1 includes acquisition means 11, acquisition means 12, storage means 13, determination means 14, addition means 15, output means 16, and accepting means 17. Acquisition means 11 acquires target audio. The target audio means audio that is mainly listened to in hearing training, and is, for example, music, audio of something being read aloud, voice of a conversation partner, audio of a television broadcast, or a radio broadcast. The target audio may be a reproduced recording or may be acquired in real time using a microphone. Acquisition means 12 acquires training intensity. The training intensity is an index that indicates training intensity of the hearing training, that is, how difficult it is to hear the target audio. The stronger (higher) the training intensity is, the more difficult it is to hear the target audio, and the weaker (lower) the training intensity is, the easier it is to hear the target audio. Storage means 13 stores a noise sound source to be added to the target audio. In this example, storage means 13 stores a plurality of types of noise sound sources. A noise sound source means data for generating a noise. A noise may be any sound that is different from the target audio, and is, for example, a natural sound such as the sound of sea waves or a sound that can be heard in the mountains, or an artificial sound such as a human voice or a so-called white noise. Determination means 14 determines an attribute of a noise to be added to the target audio according to a provided training intensity. Attributes of the noise include, for example, at least one of the type of the noise sound source, volume, a relative playback speed relative to the target audio, and the number of noise sound sources. It can also be said that the determination means 14 is a change means that changes an attribute of the noise. Addition means 15 adds a noise that has an attribute determined by determination means 14 to the target audio acquired by acquisition means 11. Output means 16 outputs the target audio together with the noise added to the target audio by addition means 15. Accepting means 17 accepts feedback from a user regarding the target audio output by output means 16. The feedback from the user means input of a subjective view of the user regarding the training, and is, for example, input of information that identifies a part where it is difficult to hear the target audio or a part where it is easy to hear the target audio.

FIG. 2 is a diagram illustrating an example of a hardware configuration of hearing training apparatus 1. Hearing training apparatus 1 is a computer apparatus that includes central processing unit (CPU) 101, memory 102, storage 103, touch screen 104, microphone 105, and headphone 106, and more specifically, is a smartphone, for example. CPU 101 controls other hardware elements by executing programs. Memory 102 is a storage apparatus that functions as a work area when CPU 101 executes programs, and includes a random access memory (RAM), for example. Storage 103 is a non-volatile storage apparatus that stores various programs and data, and includes a solid state drive (SSD), for example. Touch screen 104 is an apparatus that has both a function as a display apparatus and a function as an input apparatus, and includes a liquid crystal display (LCD) and a touch sensor provided on the LCD, for example. Microphone 105 is an apparatus that collects ambient sound and converts it into a sound signal. Headphone 106 is an apparatus that outputs a sound corresponding to a sound signal.

In this example, storage 103 stores a program (hereinafter referred to as a "hearing training program") for causing the computer apparatus to function as hearing training apparatus 1. As a result of CPU 101 executing the hearing training program, the functions illustrated in FIG. 1 are realized by the computer apparatus. CPU 101 executing the hearing training program is an example of acquisition means 11, acquisition means 12, determination means 14, and addition means 15. At least one of memory 102 and storage 103 is an example of storage means 13. Headphone 106 is an example of output means 16. Touch screen 104 is an example of accepting means 17.

### 1-2. Operation

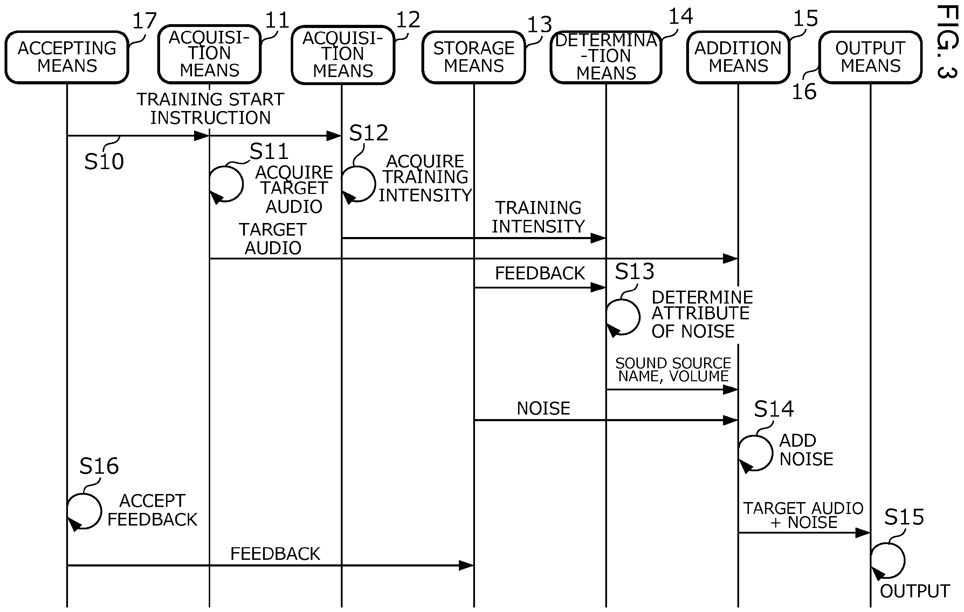

FIG. 3 is a sequence chart illustrating an example of operations of hearing training apparatus 1. The flow of FIG. 3 is started when, for example, an instruction to start up the hearing training program is received. The following describes functional elements such as acquisition means 11 as the subjects of processing. This means that a hardware element (CPU 101 or the like) that executes software (hearing training program) executes the processing in cooperation with other hardware elements.

In step S10, accepting means 17 accepts an instruction to start training. The instruction to start training is accepted through a predetermined UI screen.

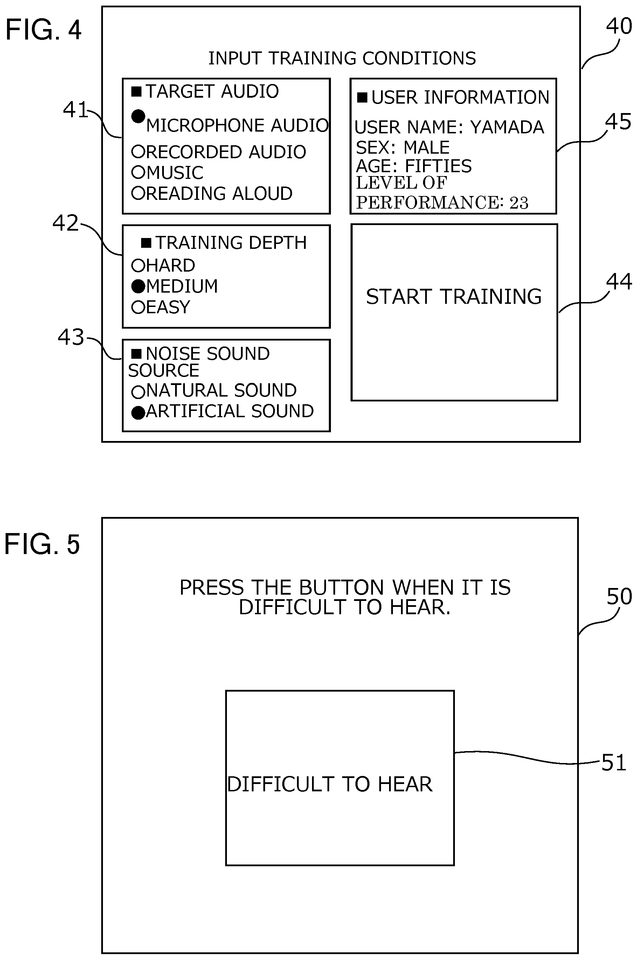

FIG. 4 is a diagram illustrating an example of UI screen 40 for accepting the instruction to start training. Accepting means 17 displays UI screen 40. UI screen 40 includes image objects 41 to 45. Image object 41 is an image object for selecting target audio. In this example, "microphone audio" and "recorded audio" are provided as options of the target audio. The microphone audio is audio that is input in real time through microphone 105. The recorded audio is audio that is stored as data in storage means 13. Further, "music" and "reading aloud" are provided as options of the recorded audio. Image object 42 is an image object for specifying training depth. The training depth is an index that indicates the depth of training, and relates to the training intensity. The deeper (hard) the training depth is, the more difficult it is to hear the target audio, and the shallower (easy) the training depth is, the easier it is to hear the target audio. In this example, the following three levels "hard", "medium", and "easy" are provided as options of the training depth. Image object 43 is an image object for selecting a noise sound source. In this example, "natural sound" and "artificial sound" are provided as options of the noise sound source. Image object 44 is an image object for inputting an instruction to start the hearing training, and more specifically, is a start button. Image object 45 is an image object that shows information regarding attributes of the user. In this example, a user name, an accumulated training time, and a level of performance are shown as the attributes of the user. The accumulated training time indicates an accumulated value of time periods of training that has been performed using the hearing training program. The level of performance is set as an index that indicates training progress, and increases as the training progresses. The user inputs, for example, an instruction to select target audio by touching positions on touch screen 104 corresponding to these image objects. When the user presses the start button of image object 44, accepting means 17 outputs a training start instruction to acquisition means 11 and acquisition means 12.

The following refers to FIG. 3 again. When the training start instruction is input, acquisition means 11 acquires target audio (step S11). The training start instruction includes information that is input through UI screen 40 and identifies target audio. Acquisition means 11 acquires the target audio in accordance with this information. If "microphone audio" is selected on UI screen 40, acquisition means 11 acquires a sound signal that is input from microphone 105 as the target audio. If "recorded audio" is selected on UI screen 40, acquisition means 11 reads out data that is stored in storage means 13 and acquires a sound signal obtained by decoding this data as the target audio. Acquisition means 11 outputs the sound signal of the target audio to addition means 15.

When the training start instruction is input, acquisition means 12 acquires training intensity (step S12). Acquisition means 12 acquires the training intensity using information input through UI screen 40. Specifically, acquisition means 12 calculates the training intensity using the training depth, the level of performance, and a predetermined mathematical formula. The training intensity calculated using this mathematical formula increases as the level of performance increases and as the training depth increases, for example. Acquisition means 12 acquires the training intensity through this calculation. Acquisition means 12 outputs information that indicates the acquired training intensity to determination means 14.

In step S13, determination means 14 determines attributes of the noise. In this example, the attributes determined in step S13 are a noise sound source name and volume. The noise sound source name is identification information for identifying a noise sound source data piece to be used out of a plurality of noise sound source data pieces stored in storage means 13. The volume indicates relative volume of the noise relative to volume of the target audio. For example, in the case where the target audio is microphone audio, the relative volume of the noise means a ratio of a signal level of the noise to an input level of a sound signal that is input through microphone 105. In the case where the target audio is recorded audio, the relative volume of the noise means a ratio between the signal level of the noise and an average signal level of a sound signal of the recorded audio. Determination means 14 first determines the noise sound source name. Determination means 14 selects the noise sound source name according to the training intensity acquired by acquisition means 12. In the example of FIG. 4, what is input through image object 43 is only information that specifies the category of the noise sound source (natural sound or artificial sound), and does not specifically identify a noise sound source name. In this case, determination means 14 selects one noise sound source data piece from among a plurality of noise sound source data pieces that belong to the specified category according to a predetermined algorithm, and determines the use of the selected noise sound source. Specifically, the selection is performed as follows. For example, storage means 13 stores a table in which noise sound source data pieces and corresponding training intensities are recorded. Determination means 14 selects one noise sound source data piece from among noise sound source data pieces that have the training intensity acquired by acquisition means 12.

Next, determination means 14 determines the volume of the noise. Determination means 14 uses the training intensity and attribute information regarding the user as information for determining the volume. Here, feedback from the user is particularly used as the attribute information regarding the user. Although details will be described later, the feedback means information that indicates whether the target audio includes a part that is difficult to hear, and in this example, means information that identifies a part that the user specified as a "part that is difficult to hear" when the hearing training was previously performed using the same data as the currently selected noise sound source data. In this example, the volume includes information regarding a time axis, and can be changed corresponding to a playback position (playback time) of the noise sound source. For example, determination means 14 determines a reference volume according to the training intensity. Further, determination means 14 sets a volume that is lower than the reference volume for a region on the time axis that is identified as the "part that is difficult to hear" based on the feedback from the user. Determination means 14 outputs information that identifies the determined noise sound source name and volume to addition means 15.

In step S14, addition means 15 adds the noise to the target audio. Specifically, addition means 15 reads out the noise sound source data that is identified using the determined noise sound source name from storage means 13 and decodes the noise sound source data to obtain a sound signal of the noise. Addition means 15 adjusts the amplitude of the sound signal of the noise according to the input volume. Addition means 15 adds the sound signal of the noise with the adjusted amplitude to the sound signal of the target audio. If the duration of the noise is shorter than the duration of the target audio, addition means 15 repeatedly uses the sound signal of the noise from where it starts. Addition means 15 outputs the sound signal of the target audio with the noise added thereto to output means 16.

In step S15, output means 16 outputs the target audio. That is, a sound corresponding to the sound signal of the target audio with the noise added thereto is output from headphone 106. While the sound signal of the target audio is being output, accepting means 17 accepts feedback from the user (step S16).

FIG. 5 is a diagram illustrating an example of UI screen 50 for accepting feedback. While the target audio with the noise added thereto is being output from headphone 106, accepting means 17 displays UI screen 50. UI screen 50 includes image object 51. Image object 51 includes a button for specifying a part that is difficult to hear. The user operates UI screen 50 while listening to the target audio, and presses the button of image object 51 when the user feels it is difficult to hear the target audio. Accepting means 17 stores, in storage means 13, information that indicates whether the button of the image object is pressed (on) or not (off) with respect to a playback position of the noise. If the duration of the noise is equal to or longer than the duration of the target audio, a playback position of the noise is equivalent to a playback position (relative time) of the target audio.

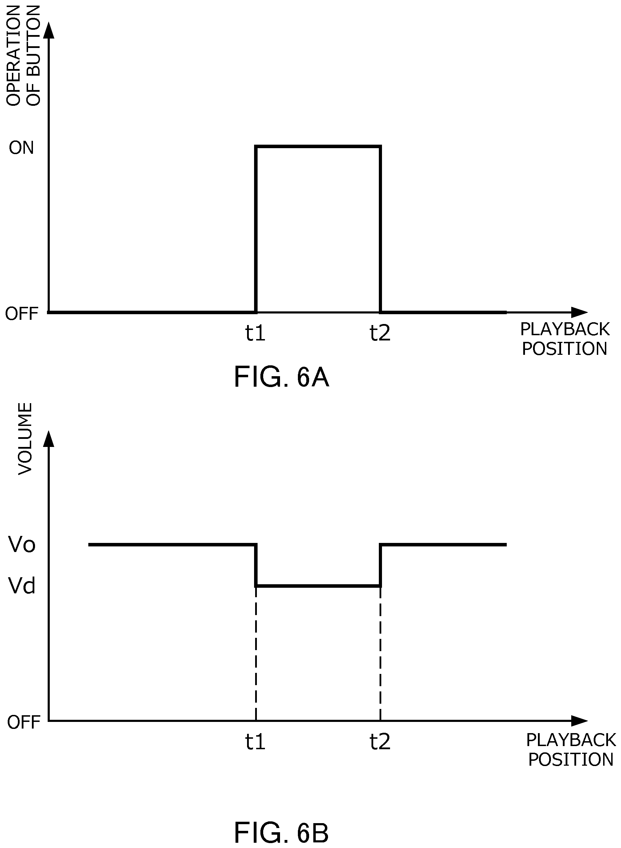

FIGS. 6 are diagrams illustrating an example of the feedback from the user. FIG. 6(A) shows feedback stored in storage means 13, and the horizontal axis represents playback position of the noise while the vertical axis represents on and off states of the button. In this example, it is shown that the button is in the on state from time t1 to time t2, and is in the off state during other periods. This feedback is used for determining the volume in step S13. FIG. 6(B) shows the volume determined in accordance with the feedback of FIG. 6(A), and the horizontal axis represents playback position of the noise while the vertical axis represents the volume. A volume Vd (< Vo) is set during the period from time t1 to time t2 relative to a reference volume Vo.

According to the present embodiment, attributes of a noise are determined according to a provided training intensity, and various noises are added to the target audio according to the training intensity, as described above. For example, even if the same target audio and the same noise sound source are used, the volume of the noise increases as the level of performance increases, and thus the training gradually becomes more difficult. In another example, even if the same target audio is used, a natural sound is used as the noise when the level of performance is low, and as the level of performance increases, an artificial sound is used as the noise, and thus the training gradually becomes more difficult.

### 2. Second Embodiment

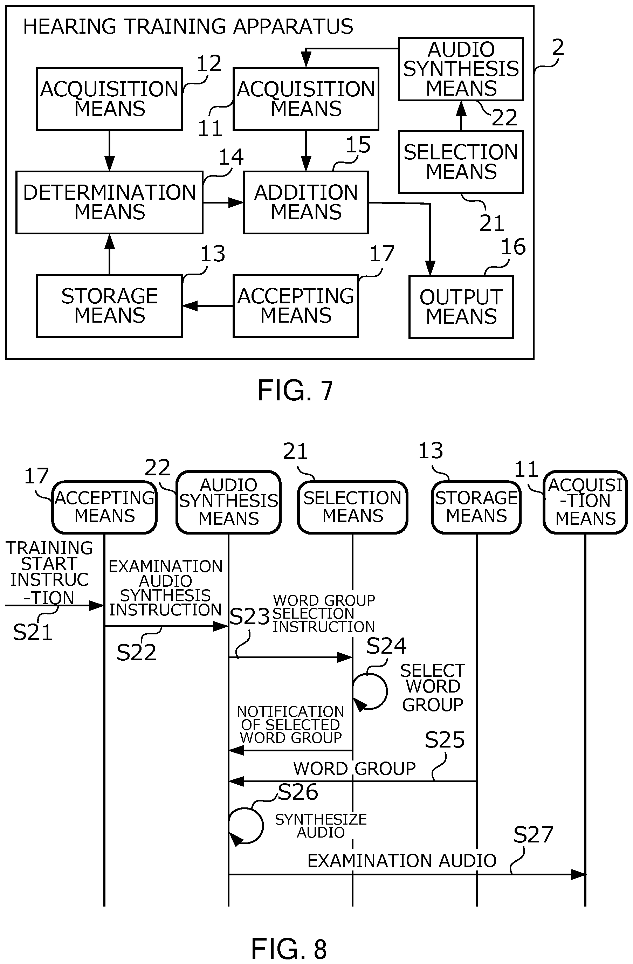

FIG. 7 is a diagram illustrating an example of a functional configuration of hearing training apparatus 2 according to a second embodiment. Hearing training apparatus 2 can be interpreted as a particular example of hearing training apparatus 1 according to the first embodiment in which target audio is more limited. In the following description, target audio in the second embodiment will be specifically referred to as "examination audio". The examination audio is audio in which sounds that belong to a certain sound group (hereinafter referred to as a "correct sound group") are arranged in order on a time axis (arranged in a time sequence). However, the examination audio sometimes includes a sound that belongs to a sound group (hereinafter referred to as a "wrong sound group") different from the correct sound group. A sound included in the correct sound group will be referred to as a correct sound, and a sound included in the wrong sound group will be referred to as a wrong sound. In an example, each sound included in a sound group is obtained by synthesizing audio in which a character string (for example, the name of an object) is spoken. The user concentrates on listening to the examination audio to judge which sound is the correct sound and which sound is the wrong sound. This increases the effects of training hearing. Note that the following omits a description of matter that is or may be common to the first embodiment.

Hearing training apparatus 2 includes selection means 21 and audio synthesis means 22. Storage means 13 stores material for generating examination audio. In the case where the examination audio is audio in which character strings (for example, the names of objects) are spoken, for example, the material is a group of words that represent the names of objects. That is, storage means 13 stores a plurality of words that belong to a plurality of word groups. Each word represents the name of an object. Attributes are allocated to the respective word groups, and the plurality of words are classified into the plurality of word groups according to attributes.

Selection means 21 selects a correct word group and a wrong word group from among the plurality of word groups. Audio synthesis means 22 synthesizes audio in which sounds of spoken words selected from the correct word group and the wrong word group are arranged in order on the time axis. This audio serves as the examination audio. A sound of a spoken word that belongs to the correct word group is a correct sound, and a sound of a spoken word that belongs to the wrong word group is a wrong sound. Storage means 13 stores audio fragment data for synthesizing audio, and audio synthesis means 22 synthesizes the examination audio using this audio fragment data. Accepting means 17 accepts input of an answer to the examination audio. The answer in the second embodiment can be interpreted as an example of the feedback in the first embodiment.

FIG. 8 is a sequence chart illustrating an example of examination audio synthesis processing according to the second embodiment. In step S21, accepting means 17 accepts an instruction to start training. The instruction to start training is accepted through a predetermined UI screen.

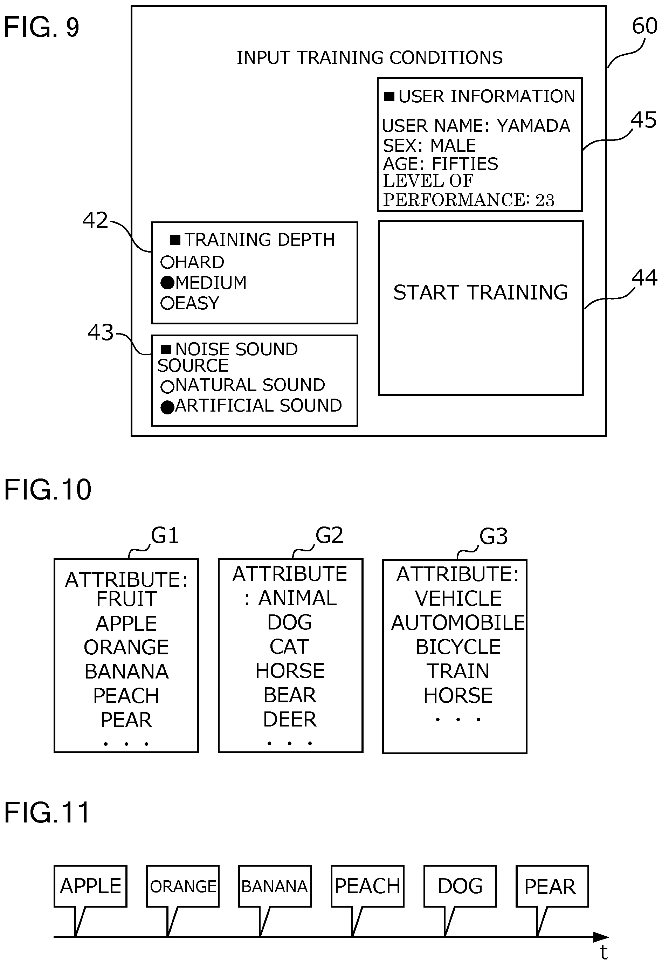

FIG. 9 is a diagram illustrating an example of UI screen 60 for accepting the instruction to start training. Accepting means 17 displays UI screen 60. UI screen 60 differs from UI screen 40 (FIG. 4) in that UI screen 60 does not include image object 41. That is, image object 41 is not necessary because the examination audio is used as the target audio in the second embodiment. Note that UI screen 60 may include an image object for specifying an attribute of the correct sound group, for example.

The following refers to FIG. 8 again. Upon accepting the instruction to start training, accepting means 17 instructs audio synthesis means 22 to synthesize the examination audio (step S22). In response to this instruction to synthesize the examination audio, audio synthesis means 22 instructs selection means 21 to select a correct word group and a wrong word group to be used for the examination audio from among a plurality of word groups stored in storage means 13 (step S23). In response to this instruction, selection means 21 selects a correct word group and a wrong word group (step S24).

FIG. 10 is a diagram illustrating an example of the plurality of word groups. Here, three word groups G1 to G3 are shown as examples. An attribute "fruit" is allocated to word group G1. The names of fruits ("apple", "orange", "banana", and the like) belong to word group G1. An attribute "animal" is allocated to word group G2. The names of animals ("dog", "cat", "horse", and the like) belong to word group G2. An attribute "vehicle" is allocated to word group G3. The names of vehicles ("automobile", "bicycle", "train", "horse", and the like) belong to word group G3. One word might belong to a plurality of different word groups (in the example of FIG. 8, the word "horse" belongs to both word group G2 and word group G3), because words can be classified according to various points of view.

The following refers to FIG. 8 again. Audio synthesis means 22 reads out the correct word group and the wrong word group from storage means 13 (step S25). Audio synthesis means 22 synthesizes the examination audio using the correct word group and the wrong word group (step S26). Specifically, the examination audio is synthesized as described below, for example. Audio synthesis means 22 generates a word string by selecting each word from the correct word group and the wrong word group. An algorithm for selecting words from the correct word group and the wrong word group and a ratio of wrong words included in the word string are defined in the hearing training program. For example, the number of correct words included in the word string is larger than the number of wrong words included in the word string. Further, a correct word is always interposed between two wrong words that are close to each other in the word string, so that the word string does not include two wrong words that are next to each other. Further, if a word belongs to both the correct word group and the wrong word group, the word is not used for the examination audio or is used only as a correct word. Audio synthesis means 22 synthesizes audio in which this word string is spoken. Storage means 13 stores an audio fragment database to be used for audio synthesis, and audio synthesis means 22 synthesizes the audio using this audio fragment database. The audio fragment database is a database in which vocal sound fragments are recorded for each speaker. Vocal sound fragments of a plurality of speakers may be recorded in the audio fragment database, in which case, audio synthesis means 22 selects a speaker in accordance with an instruction from the user or a predetermined algorithm.

FIG. 11 is a diagram illustrating an example of the examination audio. In this example, word group G1 is the correct word group and word group G2 is the wrong word group. Words included in these word groups are spoken in order at intervals. Specifically, audio in which "apple", "orange", "banana", "peach", "dog", and "pear" (out of which "dog" is a wrong sound and the others are correct sounds) are spoken is generated. Audio synthesis means 22 outputs the generated examination audio to acquisition means 11 (step S27). That is, acquisition means 11 acquires the examination audio from audio synthesis means 22. Subsequent processing is basically common to the sequence of FIG. 3.

However, feedback that accepting means 17 accepts in the present embodiment is an answer to the examination audio. The answer to the examination audio is information regarding wrong sounds, and includes at least one of the following (1) to (3), for example.

### (1) The number of wrong sounds

Accepting means 17 accepts input of the number of wrong sounds as the answer to the examination audio. In the example of FIG. 11, the number of wrong sounds is one.

### (2) The character string representing a wrong sound

Accepting means 17 accepts input of the number of wrong sounds as the answer to the examination audio. In the example of FIG. 11, the character string "dog" is input.

### (3) The time at which a wrong sound is output

Accepting means 17 displays a UI screen (not illustrated) that includes a button on touch screen 104. While listening to the examination audio, the user presses this button when the user judges that a wrong sound has been output.

Note that the answer may be a combination of two or more of (1) to (3). For example, accepting means 17 may accept input of the number of wrong sounds and character strings representing the wrong sounds.

Note that output means 16 may give the user a hint for identifying wrong sounds before outputting the examination audio. This hint indicates, for example, the attribute of the wrong sound group (for example, "animals are included among fruits") or the number of wrong sounds (for example, "there are three wrong sounds").

Attributes of the noise are changed according to the training intensity as described in the first embodiment. In the second embodiment, an attribute of correct sounds or wrong sounds may be changed in addition to or instead of attributes of the noise. That is, in the second embodiment, determination means 14 determines an attribute of at least one of the examination audio and the noise added to the examination audio according to a provided training intensity. In the case where an attribute of the examination audio is changed, the attribute is determined before audio synthesis.

In an example, an attribute of the examination audio that is changed is a fundamental frequency (pitch) of audio in which a wrong sound is spoken, for example. In this example, as the training intensity increases, audio synthesis means 22 sets the frequency of audio in which a sound belonging to the wrong sound group is spoken to be higher than the frequency of audio in which a sound belonging to the correct sound group is spoken. For some people, it is difficult to hear a sound in a high frequency band. Accordingly, if a wrong sound is output as a sound in such a frequency band that is difficult to hear, it is possible to make the user concentrate on the examination audio and judgement of the wrong sound. Note that the frequency band in which the user feels it is difficult to hear a sound may be identified in advance through measurement. Also, as for a user who feels it is difficult to hear a sound in a relatively low frequency, the frequency of audio in which a sound belonging to the wrong sound group is spoken may be set lower than the frequency of audio in which a sound belonging to the correct sound group is spoken, as the training intensity increases. The degree of difficulty of the training can be adjusted by adjusting the frequency of the examination audio as described above.

In another example, an attribute of the examination audio that is changed is the speaker who says wrong sounds. In this example, audio synthesis means 22 synthesizes audio of correct sounds and audio of wrong sounds using audio fragment data of different speakers. Audio synthesis means 22 uses different types of speakers as the training intensity increases. For example, when the training intensity is lower than a threshold, audio synthesis means 22 synthesizes both correct sounds and wrong sounds using audio fragment data of child speakers, and when the training intensity is higher than the threshold, synthesizes correct sounds using audio fragment data of an adult speaker and synthesizes wrong sounds using audio fragment data of a child speaker. Note that this is merely an example, and when the training intensity is higher than the threshold, correct sounds may be synthesized using audio fragment data of a child speaker and wrong sounds may be synthesized using audio fragment data of an adult speaker. Alternatively, correct sounds and wrong sounds may be distinguished using a male speaker and a female speaker, or using a young speaker and an aged speaker. The difficulty of the training can be adjusted by properly using different speakers as described above.

In another example, an attribute of the examination audio that is changed is the ratio of wrong sounds included among correct sounds. In this example, audio synthesis means 22 decreases the ratio of included wrong sounds as the training intensity increases. In another example, an attribute of the examination audio that is changed is a distance between the correct sound group and the wrong sound group. The position of each sound group is defined in a semantic space in which meanings of sounds are converted into numerical values. Selection means 21 selects two sound groups that are close to each other as the correct sound group and the wrong sound group as the training intensity increases.

In the above-described example, a sound of a spoken word belonging to the correct word group is used as a correct sound, and a sound of a spoken word belonging to the wrong word group is used as a wrong sound. However, the correct sound and the wrong sound are not limited as such. For example, one word group may be used for the examination audio, and correct sounds and wrong sounds may be distinguished using different speakers. In this case, when the training intensity is low, speakers who largely differ from each other in voice type may be selected, such as the voice of an adult man being used for correct sounds and the voice of a girl being used for wrong sounds, and as the training intensity increases, speakers who are close to each other in voice type may be selected, such as the voice of an adult man being used for correct sounds and the voice of another adult man being used for wrong sounds. In another example, correct sounds and wrong sounds may be sounds that are prepared as data in advance rather than synthesized audio in which words are spoken. The sounds may be, for example, sounds of musical instruments (for example, the sound of a trumpet is the correct sound and the sound of a saxophone is the wrong sound) or engine sounds of automobiles (the sound of an engine of an automobile from a particular manufacturer is the correct sound and the sound of an engine of an automobile from another manufacturer is the wrong sound).

### 3. Variations

The present invention is not limited to the above-described embodiments, and can be carried out with variations. The following describes some variations. Two or more features of the first and second embodiments and the following variations may be used in combination.

Attributes of the noise that are changed according to the training intensity are not limited to those described in the embodiments. In addition to or instead of the noise sound source name and volume, other attributes may be changed. An attribute that is changed may be, for example, a relative playback speed of the noise relative to the target audio. For example, determination means 14 increases the playback speed of the noise as the training intensity increases, and decreases the playback speed as the training intensity decreases. Alternatively, an attribute that is changed may be the number of noise sound sources. For example, determination means 14 increases the number of noise sound sources as the training intensity increases, and decreases the number of noise sound sources as the training intensity decreases. Determination means 14 may change a plurality of attributes of the noise in combination.

An attribute of the user that determination means 14 uses to determine attributes of the noise is not limited to feedback from the user. Information such as sex, name, age, hometown, or medical history of the user may be used instead of or in addition to the feedback from the user. Alternatively, determination means 14 may determine attributes of the noise according to the training intensity only, without using attributes of the user.

The method for acquiring the training intensity is not limited to that described in the embodiments. For example, acquisition means 12 may acquire the level of performance itself as the training intensity. In this case, the training intensity is automatically determined by the hearing training program without input being made by the user. Alternatively, acquisition means 12 may acquire the training depth itself as the training intensity. In this case, the training intensity is determined based on input made by the user.

Output means 16 is not limited to a means that outputs the target audio and the noise that are added as sound signals to each other from the same headphone. For example, output means 16 may include a plurality of speaker units and output the target audio from one speaker unit and output the noise from another speaker unit. In this case, the target audio and the noise are not added to each other as sound signals.

The functional configurations of hearing training apparatus 1 and hearing training apparatus 2 are not limited to those illustrated in FIGS. 1 and 7. Some of the functions shown in FIG. 1 or 7 may be omitted or a function that is not shown in FIG. 1 or 7 may be added. Also, the hardware configuration of hearing training apparatus 1 and hearing training apparatus 2 is not limited to that illustrated in FIG. 2. Hearing training apparatus 1 and hearing training apparatus 2 may have any hardware configuration so long as required functions can be realized. For example, hearing training apparatus 1 may include a speaker instead of headphone 106. Also, a plurality of apparatuses may physically cooperate with each other to function as hearing training apparatus 1. In this case, some functions of hearing training apparatus 1 may be realized by a server in a network.

The UI screens described in the embodiments are merely examples, and UI screens used in hearing training apparatus 1 are not limited to those described above. Also, the types of the target audio and noise sound sources, levels of training depth, user information, and the like described in the embodiments are merely examples, and the present invention is not limited by these examples.

The method for determining the volume according to feedback from the user is not limited to that described in the embodiments. The embodiments are described regarding a case where a volume that is lower than the volume in other parts is set for a part of a time region of the noise sound source for which the user inputs feedback indicating that it is difficult to hear. However, in a situation in which the relative volume of the noise is a certain value (first value), if feedback indicating that the target audio is difficult to hear in a part of the time region is input, determination means 14 may decrease the volume of the entire noise sound source when the training is next provided to the user.

The intended use of the hearing training apparatus of the present invention is not limited to improvement of hearing or prevention of dementia, and the hearing training apparatus may be used for any other purpose so long as it is used for stimulating the brain through hearing. For example, the ultimate goal of the use of the hearing training apparatus may be fixing memories by stimulating the brain through hearing. Specifically, the hearing training apparatus may be used for fixing memories of what is learned in various classes, seminars, or lectures, by using audio recorded in classes or the like as the target audio.

The hearing training program may be provided in the form of a storage medium such as an optical disk, a magnetic disk, or a semiconductor memory, or may be downloaded via a communication line such as the Internet. Note that the hearing training program need not include all the steps of FIG. 3 and may include only some of the steps.

## Claims

1. A hearing training apparatus comprising:
an acquisition means that acquires examination audio in which sounds selected from a determined correct sound group and a determined wrong sound group are arranged in a time sequence;
a determination means that determines an attribute of at least one of the examination audio and a noise to be added to the examination audio according to a provided training intensity;
an addition means that adds a noise having an attribute determined by the determination means to the examination audio; and
an output means that outputs the examination audio together with the noise added to the examination audio by the addition means.

2. The hearing training apparatus according to claim 1, further comprising:
an accepting means that accepts input of an answer regarding a sound that belongs to the wrong sound group out of sounds presented by the examination audio.

3. The hearing training apparatus according to claim 2,
wherein the accepting means accepts, as the answer, the number of sounds that belong to the wrong sound group out of the sounds presented by the examination audio.

4. The hearing training apparatus according to claim 2,
wherein the accepting means accepts, as the answer, a character string that represents the sound belonging to the wrong sound group out of the sounds presented by the examination audio.

5. The hearing training apparatus according to claim 2,
wherein the accepting means accepts, as the answer, a time at which the sound belonging to the wrong sound group is output while the examination audio is being output by the output means.

6. The hearing training apparatus according to any one of claims 1 to 5, further comprising:
a storage means that stores audio fragments; and
an audio synthesis means that synthesizes the examination audio using the audio fragments,
wherein the acquisition means acquires the examination audio synthesized by the audio synthesis means.

7. The hearing training apparatus according to claim 6,
wherein the audio synthesis means sets a frequency of audio in which a sound belonging to the wrong sound group is spoken to be higher than a frequency of audio in which a sound belonging to the correct sound group is spoken.

8. The hearing training apparatus according to claim 6 or 7,
wherein the storage means stores audio fragments of a plurality of speakers, and
the audio synthesis means synthesizes the examination audio using audio fragments of a speaker who is selected from among the plurality of speakers according to the training intensity.

9. The hearing training apparatus according to any one of claims 6 to 8,
wherein the storage means stores a plurality of word groups each including a plurality of words,
the hearing training apparatus includes a selection means that selects a correct word group and a wrong word group from among the plurality of word groups, and
the audio synthesis means synthesizes audio in which words selected from the correct word group and the wrong word group are spoken in order on a time axis.

10. The hearing training apparatus according to any one of claims 1 to 9,
wherein the attribute determined by the determination means includes at least one of:
a noise sound source;
volume;
a relative playback speed of the noise relative to the examination audio; and
the number of noise sound sources.

11. A hearing training method, comprising:
acquiring examination audio in which sounds selected from a determined correct sound group and a determined wrong sound group are spoken in order;
determining an attribute of at least one of the examination audio and a noise to be added to the examination audio according to a provided training intensity;
adding a noise having an attribute determined by the determination means to the examination audio; and
outputting the examination audio together with the noise added to the examination audio.

12. A program that causes a computer to execute:
acquiring examination audio in which sounds selected from a determined correct sound group and a determined wrong sound group are spoken in order;
determining an attribute of at least one of the examination audio and a noise to be added to the examination audio according to a provided training intensity;
adding a noise having an attribute determined by the determination means to the examination audio; and
outputting the examination audio together with the noise added to the examination audio.
